# EUROPEAN PATENT SPECIFICATION

(11) **EP 3 760 614 B1**
(45) Date of publication and mention of the grant of the patent: **23.03.2022**
(21) Application number: 19183945.5
(22) Date of filing: 02.07.2019
(51) Int. Cl.: C07D 209/12, C07D 209/22, C07D 209/24, C07D 209/40, C07D 231/56, C07D 311/16, A61K 31/352, A61K 31/404, A61K 31/416, A61P 35/00

(54) **CHEMICAL INHIBITORS OF ID PROTEINS FOR THE TREATMENT OF CANCER AND OTHER DISEASES**
CHEMISCHE INHIBITOREN VON ID-PROTEINEN ZUR BEHANDLUNG VON KREBS UND ANDEREN ERKRANKUNGEN
INHIBITEURS CHIMIQUES DE PROTÉINES D'IDENTIFICATION POUR LE TRAITEMENT DU CANCER ET D'AUTRES MALADIES

(43) Date of publication of application: 06.01.2021
(73) Proprietor: Universität Heidelberg, 69117 Heidelberg (DE); Karlsruher Institut für Technologie, 76131 Karlsruhe (DE)
(72) Inventor: Sleeman, Jonathan, 76646 Bruchsal (DE); Sedlmeier, Georg, 68309 Mannheim (DE); Jung, Nicole, 64319 Pfungstadt (DE); Bräse, Stefan, Troisdorf 53842 (DE); Gräßle, Simone, 76571 Gaggenau (DE)
(74) Representative: Müller-Boré & Partner Patentanwälte PartG mbB

(56) References cited:
- WO-A1-2018/165569
- MA Y. ET AL.: "Facile Access to 3-Acylindoles through Palladium-Catalyzed Addition of Indoles to Nitriles: The One-Pot Synthesis of Indenoindolones", CHEMISTRY A EUROPEAN JOURNAL, vol. 19, 13 December 2012 (2012-12-13), pages 1189-1193, XP002793673,
- WANG W.-J. ET AL.: "Feasible selective synthesis of 3-Acetylindoles and 3-Acetoacetylindoles from beta-ethylthio-beta-indoly alpha, beta-unsaturated ketones", SYNTHETIC COMMUNICATIONS, vol. 49, no. 3, 29 January 2019 (2019-01-29), pages 377-385, XP002793674,
- SHAO J. ET AL.: "A straightforward synthesis of N-monosubstituted alpha-keto amides via aerobic benzylic oxidation of amides", TETRAHEDRON, vol. 68, 14 January 2012 (2012-01-14), pages 573-579, XP028336791, DOI: 10.1016/j.tet.2011.11.005
- WANG T.-T. ET AL.: "Versatile synthesis of functionalized beta- And gamma-carbolines: Via Pd-catalyzed C-H addition to nitriles/cyclization sequences", CHEMICAL COMMUNICATIONS, vol. 54, no. 16, 1 February 2018 (2018-02-01), pages 2048-2051, XP002793676,

## Description

The present invention relates to compounds that inhibit expression of Id1 and/or Id3, as well as said compounds for use in the treatment of cancer and other diseases and conditions associated with ld1 and/or ld3 expression.

The effective treatment of cancer continues to be a major unmet clinical need. In 2018, there will be around 18 million new cases of cancer worldwide, which is predicted to rise to 23.6 million new cases each year by 2030. Currently, around one in six of all deaths worldwide are due to cancer, which equates to 9.6 million deaths in 2018. It is estimated that around 90% of these deaths are caused by the direct and indirect effects of metastasis. Metastatic disease remains essentially incurable, and new treatments are urgently required. Given the scale of the problem, it is estimated that the global cancer therapeutics market will be worth $178,863 million by the year 2023.

The cancer stem cell theory states that tumor growth is driven by a subset of cancer stem cells (CSCs) that are able to self-renew, give rise to heterogeneous progeny, and initiate the growth of new tumors. Tumor cells in the non-CSC subpopulation do not possess these properties and are therefore non-tumorigenic. The existence of CSCs has a number of important ramifications, as it predicts, for example, that by targeting tumor cells with stemness properties it should be possible to effectively treat cancer. As metastasis is caused by the seeding of new tumors in different organs around the body, targeting stemness should also be a means of preventing or treating metastases, as CSCs are by definition the only cells that can initiate the growth of new tumors. Recent evidence suggests that acquisition of stemness properties by dormant tumor cells may allow them to grow out as overt metastases.

Tumor initiation *in vivo* is currently the gold standard in defining CSCs. The majority of published studies use co-injection of tumor cells with Matrigel to determine tumor initiation rates *in vivo.* In syngeneic animal models of breast cancer and melanoma, it could be shown that Matrigel, laminin and collagen all have strong and pronounced enhancing effects on tumor take rate. Gene expression profiling and subsequent validation showed that culture of tumor cells within 3D microenvironments composed of these ECM components strongly upregulated expression of the transcriptional regulators Id1 and Id3. This upregulation is accounted for at least in part by autocrine BMP signaling that is facilitated in 3D matrices by local accumulation around cells of self-produced BMPs.

ld1 and ld3 are genes that have been shown to play a pivotal role in the initiation of primary tumor and metastatic growth. They have been implicated in determining and maintaining CSC properties for several types of tumor, including glioma and colorectal cancer. Accordingly, their expression correlates with poor prognosis for many types of cancer. In addition to their role in regulating stemness properties, Id1 and Id3, either individually or together, have been implicated in promoting tumor cell invasiveness and resistance to chemotherapy.

Cancer entities with increased Id1 and/or ld3 levels include prostate cancer, B-acute lymphoblastic leukemia, non-small cell lung cancer, ovarian tumors, esophageal squamous cell carcinoma, breast cancer, and melanoma. In many cases, ld1 and ld3 are co-expressed in tumor tissues, underlining the importance to inhibit both ld proteins simultaneously.

ld1 and ld3 have also been implicated in the induction of angiogenesis and lymphangiogenesis, in part through upregulation of VEGF-A and VEGF-C, respectively. Inhibition of these genes may therefore inhibit not only tumor growth and progression through direct effects on the tumor cells themselves, but also through inhibiting angiogenesis and lymphangiogenesis. As angiogenesis and lymphangiogenesis are features of a number of diseases other than cancer, inhibition of Id1 and/or ld3 may have therapeutic impact in these contexts as well.

Id1 and ld3 are both involved in determining the differentiation status of immune cells, and are crucial factors in diseases that involve immune dysregulation. ld1 expands the myeloid-derived suppressor cell population, which inhibits dendritic cell differentiation and CD8⁺ T-cell proliferation. This generates a pro-tumor immunosuppressive immune milieu. ld3 inhibits the differentiation of T_{H}17 helper T-cells and promotes the generation of Foxp3⁺ T_{reg} cells. Foxp3⁺ T_{reg} cells suppress T-cell immunity and foster tolerance, and in the context of cancer inhibit anti-cancer immune responses. Furthermore, ld3 is required for strong TCR signals to both promote adoption of the γδ fate by T-cells and to oppose the αβ-fate outcome. Protumoral γδ T cells promote tumor progression by inducing an immunosuppressive tumor microenvironment, stimulating angiogenesis through cytokines they produce, interfering with dendritic cell effector function, and inhibiting antitumor T cell immunity via the PD-L1 pathway. Inhibition of ld1 and ld3 may therefore not only inhibit tumor growth and progression by direct effects on tumor cells, but also through suppressing a pro-tumor immune-suppressive microenvironment. These observations also indicate that inhibitors of Id1 and Id3 may have utility in the treatment of cancer in combination with immune checkpoint inhibitors.

Id1 is implicated in mediating epithelial-to-mesenchymal transition (EMT), which is considered to be a critical process in metastasis. In addition, EMT contributes to several fibrotic diseases such as pulmonary, hepatic, renal or cardiac fibrosis. Direct evidence suggests a critical role of ld1 in hepatic fibrogenesis. Inhibiting the function of Id1/Id3 has the potential to impair EMT and thereby interfere with metastases and fibrosis.

Recently, it was shown that knockdown of ld3 renders melanoma cells more sensitive to vemurafenib treatment, suggesting a possible role for ld3 in mediating adaptive therapy resistance in melanoma. Interestingly, resistance to paclitaxel treatment in nasopharyngeal carcinoma cells is mediated by activation of the Raf/MEK pathway, which results in increased ld1 levels. Combining BRAF/MEK inhibitors with ld1/ld3 inhibitors could prove useful for improved melanoma treatment.

Id1 and Id3 expression and activity has also been implicated in rare diseases. Id1 plays a central role in Castleman's disease, a rare lymphoproliferative disorder. Id3 plays a role in the development of fibrodysplasia ossificans progressiva (FOP), a rare genetic disease characterized by extraskeletal bone formation through endochondral ossification. Thus inhibition of Id1 and Id3 may have therapeutic utility for these diseases.

In normal physiology, Id1 inhibits adipogenic differentiation, and Id3 may have a similar role. Thus inhibition of Id1 and/or Id3 could be beneficial for modulating adipogenesis *in vivo,* or in the context of regulating differentiation of cultured stem cells, in particular iPSCs.

Further, Id3 has been reported to inhibit the implantation of trophoblasts into the uterine wall, and is implicated in recurrent miscarriage. Inhibition of Id3 could therefore increase fertility by promoting implantation and inhibiting miscarriage, either during natural pregnancies or as part of *in vitro* fertilization procedures.

Several strategies have been reported for targeting Id protein or gene expression. Peptide-based approaches have been used *in vitro* and *in vivo.* Difficulties with using these approaches for therapy include delivery of the molecule to target cells and the pharmacological properties of the substances. Further, small molecule inhibitors have been investigated. However, respective approaches lacked specificity, as expression of many other genes apart from Ids was also affected.

Furthermore, natural products and substances have been considered, but the specificity of the same for inhibiting ld expression has largely not been investigated. Among such substances, Cannabidiol (CBD) inhibits Id1 expression at the transcriptional level and several preclinical studies have suggested that CBD can inhibit tumor growth and metastasis. CBD-induced inhibition of primary tumor growth and lung metastasis in an orthotropic mammary carcinoma model was associated with reduced levels of Id1 in tumor tissue. Cannabidiol has a low affinity for CB1 and CB2, which explains why it is considered to be non-psychoactive. CBD inhibited Id1 expression in a glioma model, resulting in decreased invasion *in vivo* and prolonged survival of tumor-bearing mice.

However, other genes apart from ld1 are also regulated by CBD. Nevertheless, of the current approaches for therapeutic inhibition of Id proteins, CBD has emerged as the benchmark, due to its lack of psychoactive effects, its pharmacological properties, and the fact that it is well-tolerated.

Compounds that inhibit Id1 and/or Id3 are disclosed in WO2018/165569.

In view of the above, the technical problem underlying the present invention is the provision of means for the inhibition of Id1 and/or Id3 and respective uses for the treatment of diseases and conditions associated with Id1 and/or Id3 expression.

The solution to the above technical problem is achieved by the embodiments characterized in the claims.

The present invention relates to the following items:
1. A compound selected from the group consisting of the following compounds X8706, X8765, X8166, X8762, X8702, X8572, X8766, X8571, and X8035: 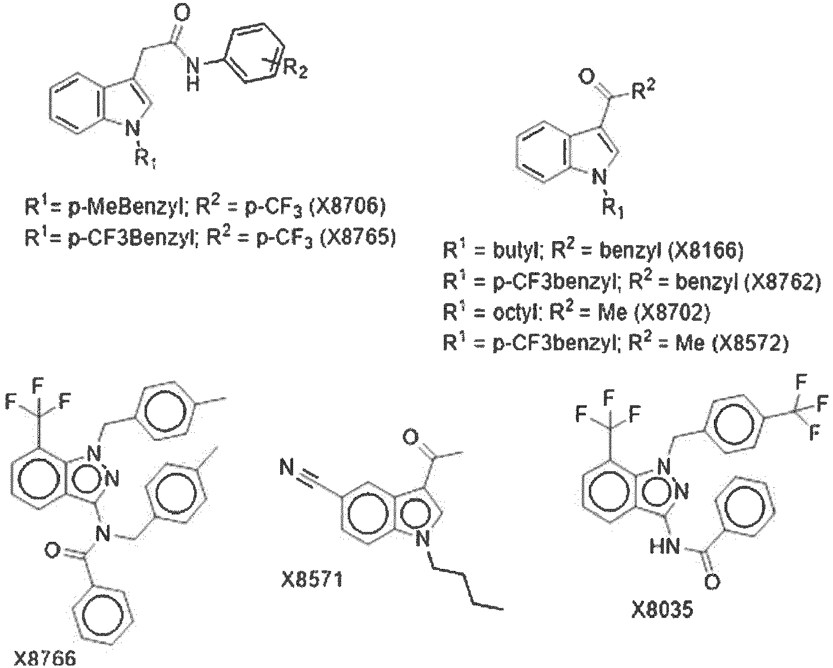
2. A compound according to item 1 for use in medicine.
3. A compound according to item 1 for use in the prevention or treatment of
   a condition or disease that is associated with cells expressing Id1 and/or Id3,
   a condition or disease that is dependent on angiogenesis, and/or
   a condition or disease that is dependent on lymphangiogenesis.
4. The compound for use according to item 3, wherein the condition or disease that is associated with cells expressing ld1 and/or ld3 is selected from the group consisting of the initiation of tumor formation, the initiation of tumor metastasis, the growth of tumors, the growth of metastases, a pro-tumor immune response, the dissemination of tumor cells, and cancer.
5. The compound for use according to item 3, wherein the condition or disease that is dependent on angiogenesis is selected from the group consisting of the initiation of tumor formation, the initiation of tumor metastasis, the growth of tumors, the growth of metastases vascular adhesion, angiofibroma, arteriovenous malformations, arthritis, atherosclerotic plaques, corneal graft neovascularization, delayed wound healing, diabetic retinopathy, granulation burns, hemangioma, hemophilic joints, hypertrophic scars, neovascular glaucoma, non-union fractures, Osler-Weber syndrome, psoriasis, progenic granuloma, retrolental fibroplasia, schleroderma, cancer, trachoma, and von Hippel-Lindau syndrome.
6. The compound for use according to item 3, wherein the condition or disease that is dependent on lymphangiogenesis is selected from the group consisting of organ transplantation, cancer, filariasis, Gorham's disease, dry eye disease, pulmonary fibrosis, inflammatory bowel disease, diabetes, chronic inflammatory diseases, chronic obstructive pulmonary disease (COPD), inflammatory arthritis, ulcerative colitis, psoriasis, and ocular surface diseases.
7. A compound according to item 1 for use in the prevention or treatment of a condition or disease, selected from the group consisting of Castleman's disease, fibrodysplasia ossificans progressiva (FOP), miscarriage, and fibrosis.
8. A compound according to item 1 for use in targeting cancer stem cells, the inhibition of angiogenesis, enhancing chemosensitivity of tumor cells, the induction of tumor cell dormancy, the maintenance of tumor cell dormancy, the inhibition of EMT (epithelial-mesenchymal transition), the suppression of VEGF-A (vascular endothelial growth factor A) expression, the suppression of VEGF-C (vascular endothelial growth factor C) expression, inducing the differentiation of stem cells and iPSCs (induced pluripotent stem cells), improving trophoblast implantation into the uterine wall, preventing an TGF-beta (transforming growth factor beta) immune-suppressive phenotype of immune cells, and/or the inhibition of myeloid-derived suppressor cells.
9. The compound for use according to any one of items 2 to 8, wherein said compound is administered in combination with one or more further compounds, selected from the group consisting of immune checkpoint inhibitors, BRAF inhibitors, and MEK inhibitors.

In particular, in a first aspect, the present invention relates to compounds according to Formula (I) of the present invention which are selected from the group consisting of the following compounds X8706, X8765, X8166, X8762, X8702, X8572, X8766, X8571, and X8035; wherein compounds X8706, X8166, X8766, and X8035 are particularly preferred; and compound X8166 is even more preferred:

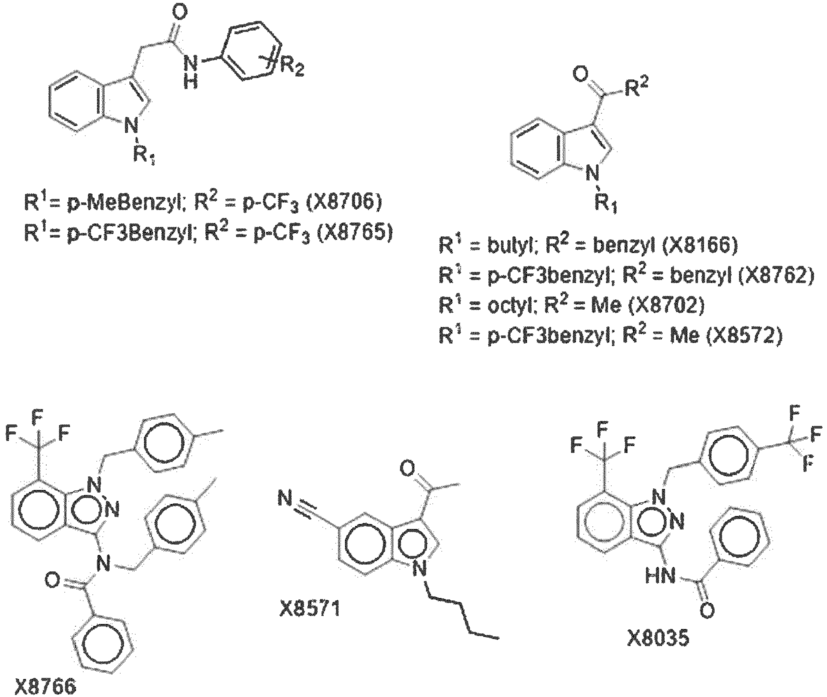

Also described herein are compounds selected from the group consisting of the following compounds X6632, X6760, X6779, X6631, X6777, X6768, X6633, X5549/X1384, X81, X106, X1312, X6945, X6910, X6404, X6770, X6778, X6758, X6944, X7776, and X7401 (being not in accordance with the claimed invention):

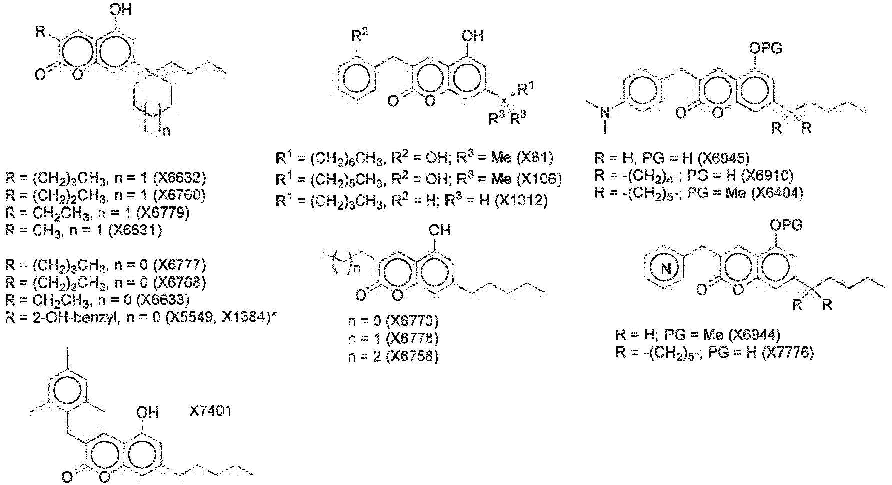

In a second aspect, the present invention relates to the compounds of the present invention for use in medicine.

In a third aspect, the present invention relates to the compounds of the present invention for use in the prevention or treatment of a condition or disease that is associated with cells expressing Id1 and/or Id3; a condition or disease that is dependent on angiogenesis, and/or a condition or disease that is dependent on lymphangiogenesis.

Conditions or diseases that are associated with cells expressing Id1 and/or Id3 can be selected from the group consisting of the initiation of tumor formation, the initiation of tumor metastasis, the growth of tumors, the growth of metastases, a pro-tumor immune response, the dissemination of tumor cells, and cancer, preferably in the context of prostate cancer, B-acute lymphoblastic leukemia, non-small cell lung cancer, ovarian tumors, esophageal squamous cell carcinoma, breast cancer, and melanoma.

Further, conditions or diseases that are dependent on angiogenesis can be selected from the group consisting of the initiation of tumor formation, the initiation of tumor metastasis, the growth of tumors, the growth of metastases, vascular adhesion, angiofibroma, arteriovenous malformations, arthritis, atherosclerotic plaques, corneal graft neovascularization, delayed wound healing, diabetic retinopathy, granulation burns, hemangioma, hemophilic joints, hypertrophic scars, neovascular glaucoma, non-union fractures, Osler-Weber syndrome, psoriasis, progenic granuloma, retrolental fibroplasia, schleroderma, cancer, trachoma, and von Hippel-Lindau syndrome.

Furthermore, conditions or diseases that are dependent on lymphangiogenesis can be selected from the group consisting of organ transplantation, cancer, filariasis, Gorham's disease, dry eye disease, pulmonary fibrosis, inflammatory bowel disease, diabetes, chronic inflammatory diseases, chronic obstructive pulmonary disease (COPD), inflammatory arthritis, ulcerative colitis, psoriasis, and ocular surface diseases.

In a fourth aspect, the present invention relates to the compounds of the present invention for use in the prevention or treatment of a condition or disease, selected from the group consisting of Castleman's disease, fibrodysplasia ossificans progressiva (FOP), miscarriage, and fibrosis.

In a fifth aspect, the present invention relates to the compounds of the present invention for use in targeting cancer stem cells, the inhibition of angiogenesis, enhancing chemosensitivity of tumor cells, the induction of tumor cell dormancy, the maintenance of tumor cell dormancy, the inhibition of EMT (epithelial-mesenchymal transition), the suppression of VEGF-A (vascular endothelial growth factor A) expression, the suppression of VEGF-C (vascular endothelial growth factor C) expression, inducing the differentiation of stem cells and iPSCs (induced pluripotent stem cells), improving trophoblast implantation into the uterine wall, preventing an TGF-beta (transforming growth factor beta) immune-suppressive phenotype of immune cells, and/or the inhibition of myeloid-derived suppressor cells.

In preferred embodiments of the compounds for use of the present invention, said compounds are administered in combination with one or more further compounds, selected from the group consisting of immune checkpoint inhibitors, BRAF (B-Raf) inhibitors, and MEK (MAPK/ERK kinase) inhibitors.

In a sixth aspect, the present disclosure (not part of the invention) relates to methods of preventing or treating any of the conditions and/or diseases as defined in the above fourth, fifth, and sixth aspects of the present invention, comprising a step of administering one or more of the compounds as defined in the first, second, and third aspects of the present invention, to a subject in need thereof. In preferred embodiments, the subject is a human subject.

The compounds of the present invention advantageously provide the dual inhibition of Id1 and Id3, a more potent inhibition of ld1 and/or ld3 as compared to known compounds, improved pharmaceutical properties as compared to known compounds, and a reduced cytotoxicity as compared to known compounds.

The figures show:
Figure 1:
   Id1 and ld3 gene ablation by CRISPR/Cas9 significantly impairs tumor growth of melanoma cells *in vivo.* Murine melanoma cells (B16-F10 or Ret) were co-injected with Matrigel into a syngeneic mouse model. (A) Single knockdown of ld1 or ld3 impairs tumor growth of Ret cells *in vivo.* (B) Simultaneous knockdown of Id1 and ld3 in B16-F10 and Ret melanoma cells significantly inhibits tumor growth *in vivo.* (C) Simultaneous knockdown of Id1 and ld3 in Ret cells is superior compared to single knockdown in inhibiting tumor growth *in vivo.* Relative tumor growth is indicated by normalization to mean value of corresponding control groups. Error bars = SEM. *p ≤ 0.05; ***p ≤ 0.001.
Figure 2:
   Loss of Id1 and ld3 results in significantly fewer colonies growing in 3D Matrigel culture. B16-F10 (left panel) or Ret (right panel) control or CRISPR/Cas9 Id1/Id3 cells were seeded in 3D Matrigel (10 mg/ml) and grown for six days before analysis. The number of colonies at six positions of each well of a 48-well plate were counted in each z-level (5-10 z-levels were imaged per position).
Figure 3:
   Example Western blot from the compound library screen of compounds structurally related to cannabidiol (CBD) for their potential to inhibit BMP4-mediated Id1 and Id3 expression in B16-F10 cells. Of the 33 compounds tested in this subset, 18 reduced Id1 and Id3 protein levels 24 h after simultaneous treatment with BMP4 (10 ng/ml) and DMSO, CBD (10 µM) or the test compounds (10 µM).
Figure 4:
   BOILED-Egg anaylsis indicates better pharmacological properties of X8166 compared to CBD. The *Brain Or IntestinaL EstimateD permeation* method *(*BOILED-Egg) predicts gastrointestinal absorption and brain access by lipophilicity (WLOGP) and polarity (tPSA) of small molecules.
Figure 5:
   X6632 and X8166 significantly inhibit melanoma growth and initiation *in vivo.* B16-F10 (A) or Ret (B) cells were co-injected with Matrigel into syngeneic mice. Mice were treated (i.p. injections) daily for the first 14 days with DMSO, CBD, X6632 or X8166. Numbers indicate the number of animals with a tumor in each group. Bar graphs show the mean tumor volume in each group at the day when the first animal had reached the legal tumor size limit. Kaplan-Meier curves show the percentage of tumor-free animals in percent as a measure for tumor initiation. Error bars = SEM. */# p=0.05; ##/**p=0.01; ###/***p=0.001. * compared to DMSO, # compared to CBD.
Figure 6:
   X8166 is less cytotoxic than CBD and X6632. (A) Loss of ld1 and ld3 expression does not significantly alter proliferation of B16-F10 and Ret cells in 2D culture. (B) Cannabidiol (CBD) is more toxic to mouse embryonic fibroblasts (MEFs) than X8166. (C) Higher concentrations of CBD are more cytotoxic in B16-F10 and Ret cells compared to X8166.
Figure 7:
   X8166 inhibits melanoma cell growth in 3D Matrigel. B16-F10 (A) or Ret (B) control or CRISPR/Cas9 Id1/Id3 cells were seeded in 3D Matrigel (10 mg/ml) and grown for six days before analysis. B16-F10 or Ret cells were treated daily with DMSO or X8166 (7.5µM). The number of colonies at six positions of each well of a 48-well plate were counted in each z-level (5-10 z-levels were imaged per position).
Figure 8:
   Analysis to determine chemical space of indole- and indazole-type compounds required for inhibition of Id1 and Id3. (A) Ret cells were treated with BMP4 and the indicated compounds (3.3 µM) for 24h prior to lysis. (B) B16-F10 or Ret cells were treated with BMP4 and the indicated compounds (10 µM) for 24h prior to lysis.
Figure 9:
   Analysis to determine chemical space of coumarine-type compounds required for inhibition of Id1 and Id3. (A) B16-F10 or Ret cells were treated with BMP4 and the indicated compounds (10 µM) for 24h prior to lysis. (B) Several compounds inhibit Id1 and Id3 expression at 3.3 µM, while CBD does not inhibit Id1 or Id3 expression at this concentration.

The present invention will be further illustrated by the following example without being limited thereto.

### Examples

### Material and methods:

### Synthetic route towards compound X6632

a) KHMDS, THF, dibromo alkane, -16 °C to r.t., 16 h, 78%; b) DIBAL-H, DCM, -78 °C, 1 h, 92%; c) 1) n-propyl triphenyl phosphonium bromide, KHMDS, THF, 0 °C to 10 °C, 30 min, 2) starting material, 10 °C, 1 h, 99%; d) Pd/C, H₂-atmosphere, ethyl acetate, 24 h, 97%; e) 1.) TMEDA, diethyl ether, 0 °C, n-BuLi, 2 h, r.t., 2.) 0 °C, DMF, 4 h, r.t., 91%; f) AlCl₃, Nal, ACN, DCM, 1.5 h, r.t., 80%; g) hexanoic acid anhydride, K₂CO₃, microwave irradiation (180 °C, 65 min, 300 W), 82%; h) BBr₃ in CH₂Cl₂ (5.00 equiv.), CH₂Cl₂, -78 °C - rt (30 min), 92% (Scheme 1).

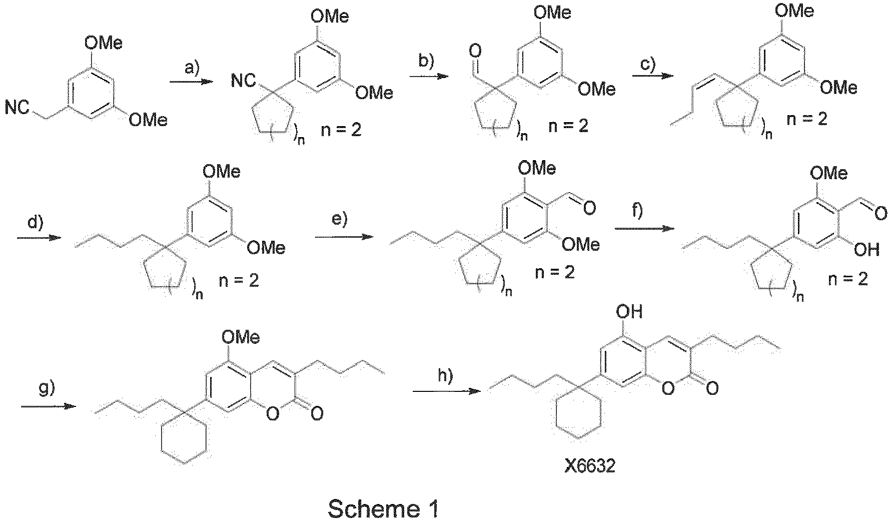

### Synthesis of compounds according to Formula lb (R¹ = butyl) (Scheme 2)

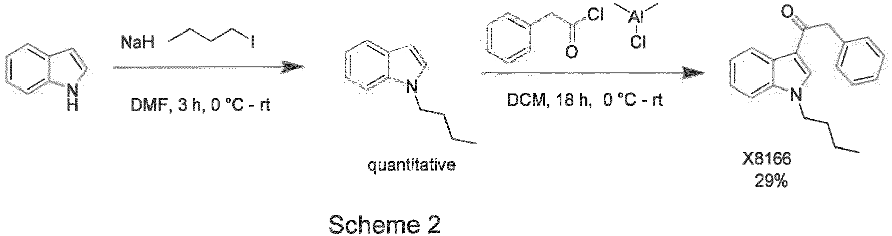

### CRISPR/Cas9

Generation of the CRISPR/Cas9 Id1/Id3 clones used in this study has been previously reported. In short, cells were co-transfected with a gRNA vector, hCas9 plasmid and sequence-specific single-stranded donor oligonucleotides using Lipofectamin2000 reagent according to the manufacturer's protocol. Single cell clones were expanded and screened for alterations in genomic DNA sequences of the Id1 and ld3 genes with sequence-specific primers. Colonies with alterations in the genomic DNA sequences were selected and checked for Id1/3 protein expression by Western blot analysis. Colonies lacking a specific band for the ld1/3 protein were selected, seeded as single cells in 96-wells and subsequently analysed for genomic alterations and the loss of Id1 and ld3 protein expression. Single cell clones obtained from the parental cell line were used as controls.

### 3D Matrigel assay

Cells (2×10³) were mixed with Matrigel to obtain 150 µl of a cell/Matrigel (10 mg/ml, #354262, Corning Inc.) solution, which was seeded into a well of a 48-well plate. The gel was allowed to solidify at 37°C for 30 min and was subsequently overlaid with 500 µl complete growth medium. After six days of culture, images were captured using a Leica DMI6000 B microscope at six different x/y positions in every well, and a minimum of five horizontal layers (z levels) at each position, in order to get every cell in focus for image analysis. Image analysis was performed using Fiji software as follows: First, images were transformed into 8-bit files (pixel values from 0 (black) to 255 (white)). The scale was set to distance=0, known=0, pixel=1, and unit=pixel global. Outlines of each colony in focus in every image were detected using the integrated semi-automatic magic wand tool. Once outlines were matching the morphology of the colony were obtained, the colony was filled white (pixel value 255) using the fill function. Thresholding of pixel values (threshold=255) allowed black (background) and white (colonies) images of the filled colonies to be obtained. The "Analyze particles" function was applied to measure the area and perimeter of colonies with a minimum pixel size of 1500. An invasive index was calculated as follows: Invasive index = (perimeter)2/area, resulting in a unitless measure of invasiveness.

### Tumor growth and initiation in vivo

Cells were harvested using trypsin/EDTA and washed in PBS. The indicated numbers of living melanoma cells were subcutaneously injected into the flank of syngeneic mice in 100 µl PBS. For co-injection experiments, the indicated number of living cells was resuspended in 100 µl Matrigel HC (10 mg/ml, #354262, Corning Inc.), and subcutaneously injected into the flank. Tumor volume was calculated using the formula 4/3π (d₁/2 × d₂/2 × d₃/2), where d₁ - d₃ represents the diameter of the tumor in three dimensions. Animals were treated for 14 days with DMSO, CBD, X6632 or X8166 (30 µl in DMSO intraperitoneal). All animal experiments were approved by the local authorities, and performed according to the German legal requirements. Tumor initiating cell (TIC) frequency and p-values were calculated using ELDA software.

### Compound screens

To screen compounds with an inhibitory effect on ld1 and ld3 expression, cells were simultaneously stimulated with BMP4 (10 ng/ml, 315-27, Peprotech) and substances at the indicated concentrations for 24 h. DMSO served as a solvent control. Effects on Id1 and ld3 expression levels were analysed by Western blotting.

### Western blot

Western blot analysis was performed using standard methods. The following antibodies were used for protein detection: Id1 (195-14, CalBioreagents), ld3 (17-3, CalBioreagents), β-actin (AC-15, Sigma Aldrich), Vinculin (VIN-11-5, Sigma Aldrich). Protein bands were detected using HRP conjugated secondary antibodies (P0447, P0448, Agilent Technologies) and enhanced chemiluminescence (32106, Thermo Fisher Scientific).

### Example 1:

### Establishment of Id1 and Id3 as targets for cancer therapy

To demonstrate a role for Id1 and ld3 in the initiation and growth of melanoma, CRISPR/Cas9 genome editing was used to permanently switch off these genes in B16-F10 and Ret melanoma cells. The tumor cells were then implanted subcutaneously into syngeneic experimental animals, and initiation and growth of melanomas was compared to controls.

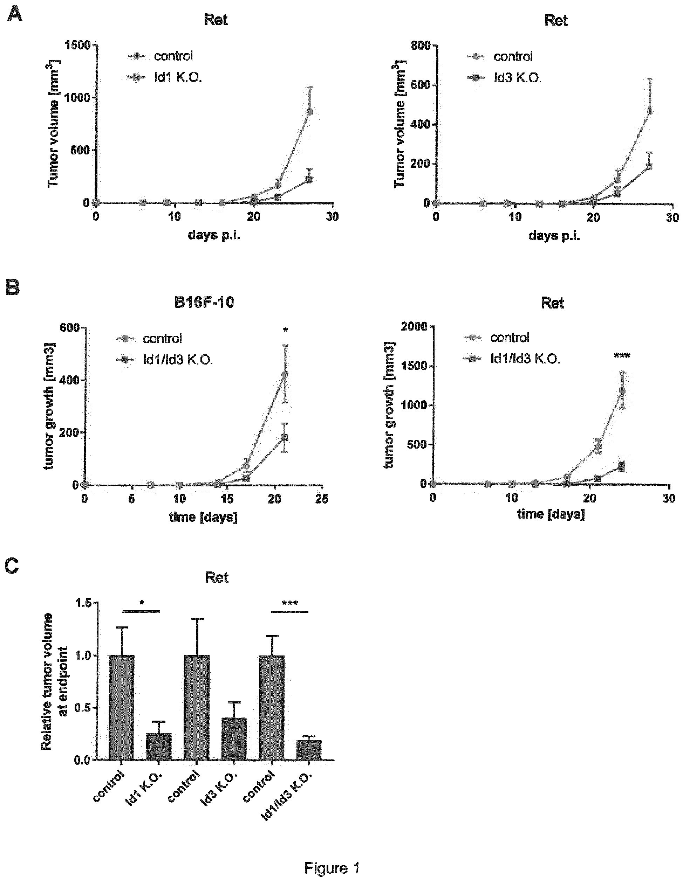

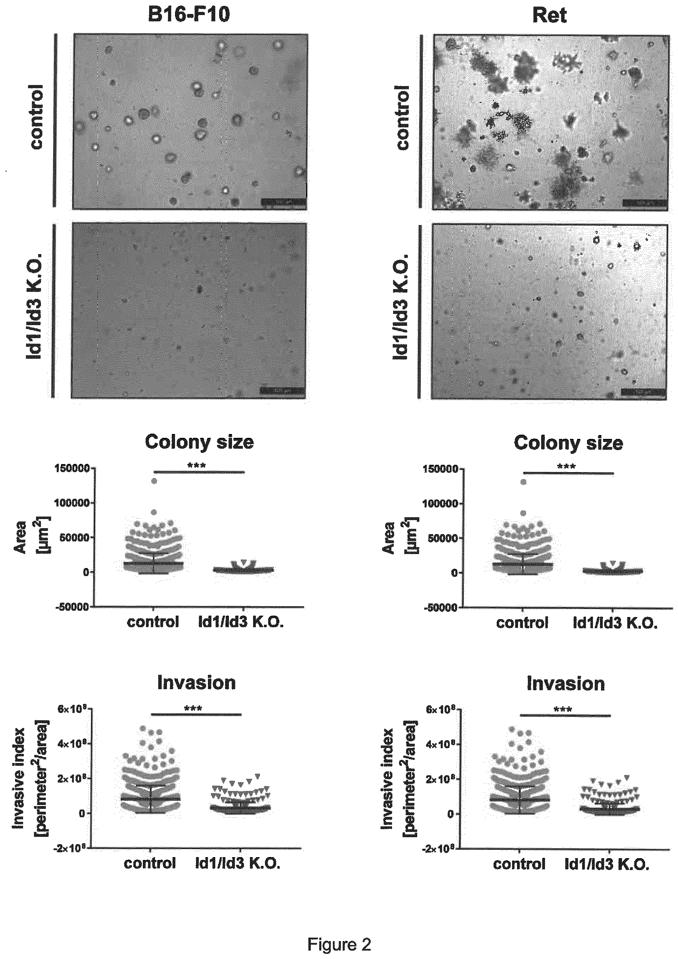

Loss of either Id1 or ld3 strongly and significantly reduced tumor growth of Ret cells *in vivo* (Fig. 1A). Simultaneous loss of Id1 and ld3 significantly inhibited tumor growth of B16-F10 and Ret cells *in vivo* (Fig. 1B), while having a more pronounced effect than single knockout cells (Fig. 1C). Although eventually all animals developed tumors, initiation of tumor growth was also significantly delayed (Table 1), consistent with a role for ld1 and ld3 in stemness properties that endow cells with enhanced tumor initiating properties. In addition, loss of Id1 and ld3 significantly impaired the outgrowth of B16-F10 and Ret cells in 3D Matrigel culture *in vitro* (Fig. 2). In Ret cells, reduced invasive behaviour was observed upon loss of ld1 and ld3 expression (Figure 2, left panel). These results indicate that targeting Id1 and ld3 should have therapeutic value.

**Table 1: Loss of Id1 and ld3 expression significantly reduces tumor initiation in vivo measured at days 28-30.**

| | | **Mice with tumors / total number of mice** | | | |
|---|---|---|---|---|---|
| **Cell line** | | **5 cells injected** | **50 cells injected** | **TIC frequency** | **p-value** |
| **B16-F10** | Control | 4/24 | 17/24 | 1/38.3 | 0.0271 |
| | Id1/Id3 K.O. | 1/24 | 11/24 | 1/85.1 | |
| **Ret** | Control | 7/24 | 20/24 | 1/24.1 | 2.06E-06 |
| | Id1/Id3 K.O. | 1/24 | 7/24 | 1/142 | |

Five or fifty B16-F10 or Ret control or CRISPR/Cas9 cells were co-injected with Matrigel (10 mg/ml) into syngeneic mice. Each group (control or CRISPR/Cas9 Id1/Id3) consisted of 24 animals (eight animals for each of the three cell clones tested). Tumor initiation was scored when tumors with a size of more than 1000 mm³ were present 28-30 days after tumor cell injection. The tumor initiation cell (TIC) frequency was measured using Extreme limiting dilution analysis (ELDA) software.

### Example 2:

### Screening of a unique chemical library identifies novel inhibitors of Id1 and Id3 protein expression

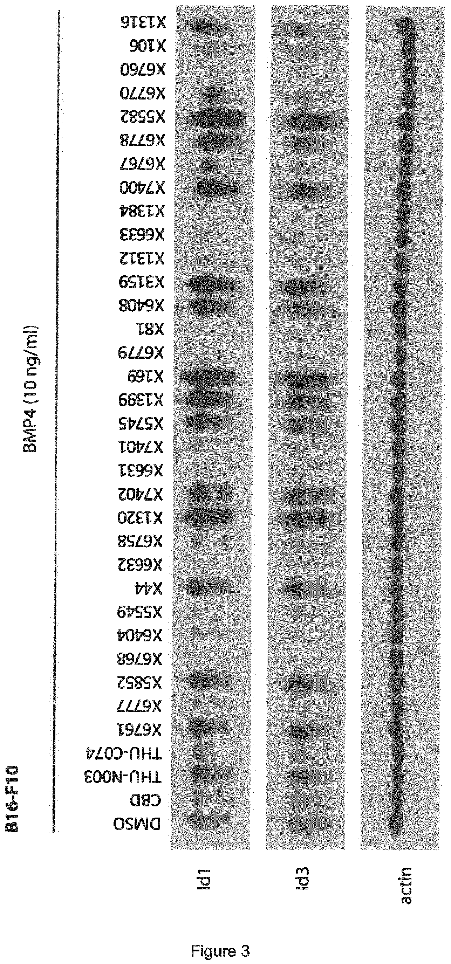

A unique chemical library containing 168 novel compounds that are structurally related to CBD was synthesized. B16-F10 and Ret melanoma cells were treated with BMP4 to induce ld1 and ld3 expression, and simultaneously treated with each of the compounds individually for a 24 h period. The ability of the compounds to inhibit Id1 and ld3 protein expression relative to CBD was assessed using Western blotting analysis. Around one third of the compounds had an inhibitory effect on both Id1 and ld3 protein expression, and 22 reduced ld1 and ld3 expression more potently than CBD (Fig. 3, Table 2). Compounds showing the strongest inhibitory activities were X81, X106, X 6632, X6760, X8035, X8166, X8706 and X8766.

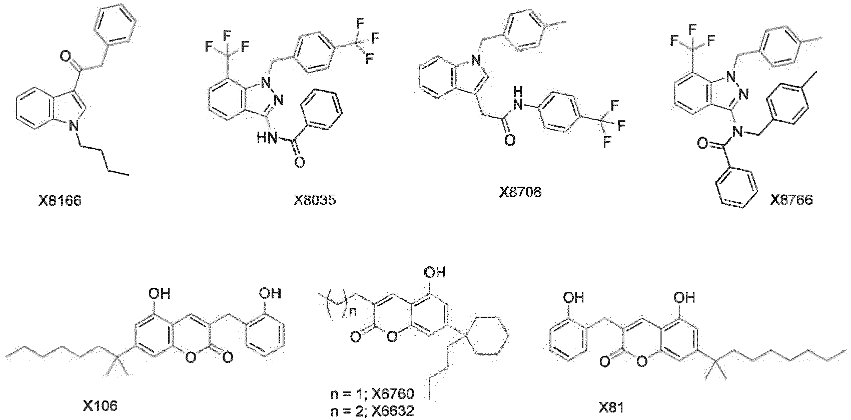

**Table 2: Summary compound screen. 168 compounds were tested for their inhibitory effect on Id1 and Id3 and rated.**

| **Total number of compounds** | **No inhibitory effect** | **Less potent than CBD** | **Similarly potent as CBD** | **More potent than CBD** |
|---|---|---|---|---|
| 168 | 106 (63.1%) | 27(16.1%) | 13 (7.7%) | 22(13.1%) |

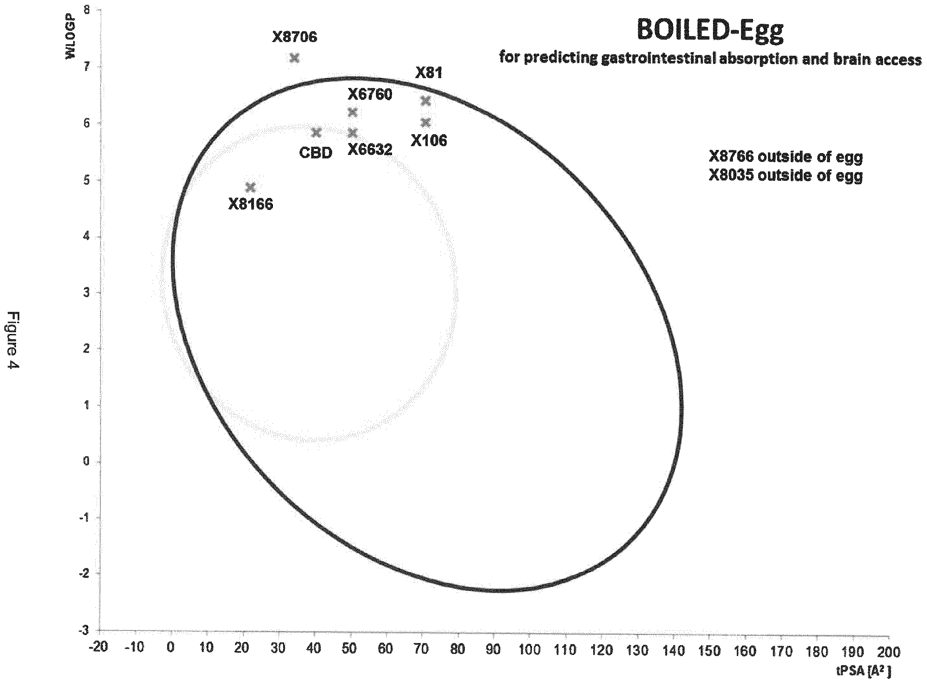

Gastrointestinal absorption and brain access are two important pharmacokinetic parameters. The *Brain Or IntestinaL EstimateD permeation* method (BOILED-Egg) predicts gastrointestinal absorption and brain access by lipophilicity (WLOGP) and polarity (tPSA) of small molecules. The BOILED-Egg analysis of the identified compounds predicts better pharmacokinetic properties of X8166 compared to CBD, while X6632 has similar properties as CBD (Fig. 4). On the basis of these predicted values and *in vitro* solubility analyses, X6632 and X8166 were investigated further in subsequent experiments.

### Example 3:

### Novel compounds that inhibit tumor growth better than CBD

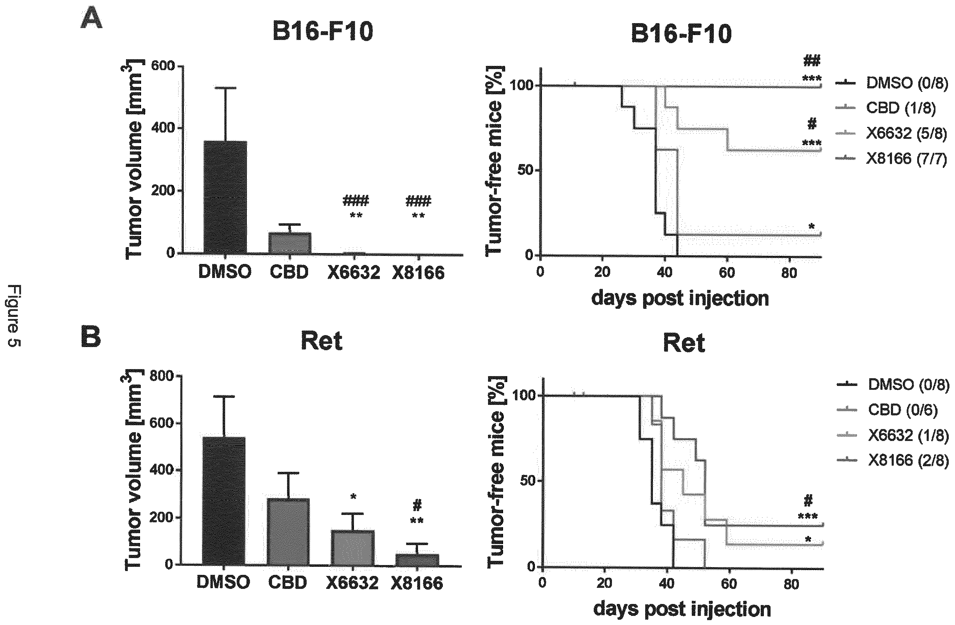

To investigate the anti-tumor properties of the selected Id1/Id3-inhibiting compounds, groups of experimental mice (8 per group) were injected subcutaneously with B16-F10 or Ret melanoma cells. DMSO as a solvent control, CBD as a reference compound, or the test substances X6632 and X8166 were injected daily into the mice for the first two weeks following implantation of the melanoma cells (Fig. 5). Melanoma initiation and growth was assessed over a period of 90 days. Compounds X6632 and X8166 significantly inhibited melanoma growth and initiation compared to DMSO treatment (Fig. 5). X8166 inhibited tumor growth and initiation significantly more compared to CBD, while X6632 was significantly more effective than CBD in the B16-F10 experiment and showed a trend towards reduced tumor initiation and growth in the Ret experiment. Importantly, none of the animals injected with B16-F10 cells and treated with X8166 grew tumors, and only three animals treated with X6632 had tumors, while all animals treated with DMSO and seven out of eight mice with CBD treatment developed tumors. When mice were injected with Ret cells and treated with X6632 or X8166, 1 mouse in the X6632 group and 2 mice in the X8166 group also did not develop any tumors. These results show that X6632 and especially X8166 have the potential to prevent tumor initiation and inhibit the growth of melanoma.

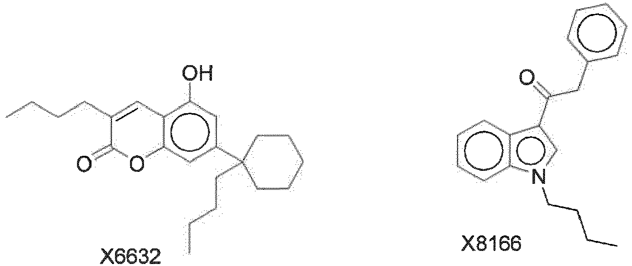

Of the tested compounds, X8166 had the best toxicology profile (no evidence for toxicity was observed for X8166 in the treated mice). In contrast, daily intraperitoneal injections of CBD led to the death of two mice in the experiment with Ret cell-derived tumors (cause of death unknown). Furthermore, when the mice were sacrificed at the end of the experiment, all CBD treated mice exhibited toxicity-related injury to their intestinal organs.

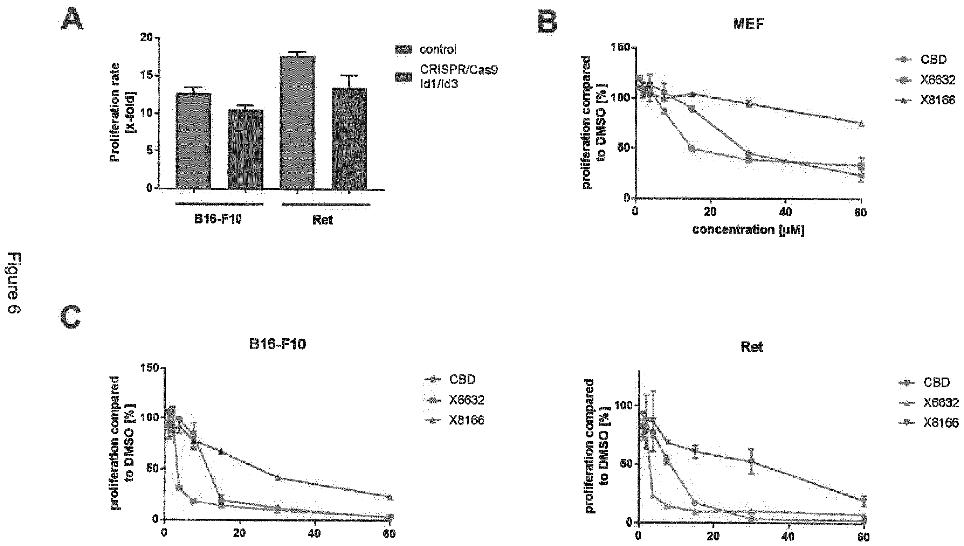

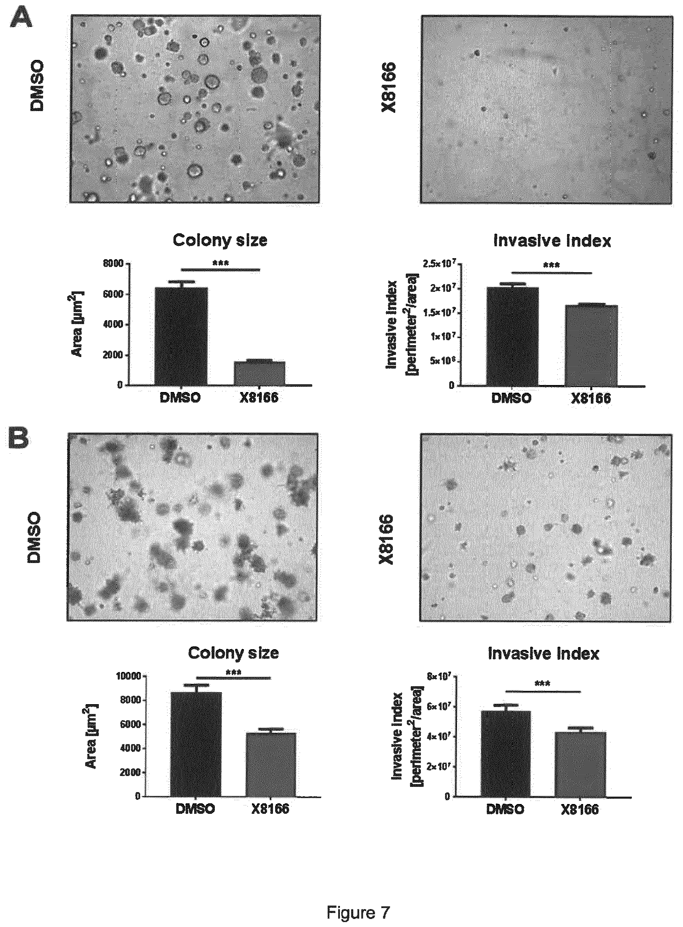

Genetic ablation of Id1 and ld3 expression by CRISPR/Cas9 genomic editing inhibited growth and invasiveness of the melanoma cells in 3D culture (Fig. 2), but did not significantly alter the proliferation of melanoma cells in standard 2D plastic culture (Fig. 6A). In 2D culture, X8166 also only had a modest inhibitory effect on the proliferation of the melanoma cells and on non-transformed mouse embryonic fibroblasts (MEF), predominantly at high concentrations (Figs. 6B, C). Similar to genetic deletion of Id1 and Id3, X8166 also significantly impaired the growth of the melanoma cells in 3D Matrigel (Fig. 7). In contrast, CBD and X6632 treatment strongly inhibited proliferation of both the melanoma cells and the MEFs in 2D culture, and 3D assays could not be performed using daily application of CBD and X6632 due to cytotoxicity. Taken together, these observations suggest that CBD and X6632 exert additional effects on melanoma cells over and above just the inhibition of ld1 and ld3 expression. Importantly, these data also indicate that X8166 has less off-target effects than the other tested substances, and phenocopies the effect of genetic deletion of ld1 and Id3, suggesting that X8166 is a much more specific inhibitor of ld1 and ld3 than the other substances.

In summary, these data identify a novel substance class that exerts superior inhibitory effects on Id1 and Id3 expression compared to CBD. It is demonstrated that X6632 and X8166 are potent inhibitors of melanoma growth and initiation in two independent syngeneic mouse models, and show that X8166 is well tolerated by mice and non-transformed cells.

### Example 4:

### Definition of the chemical space required for inhibition of Id1 / Id3 expression

Compounds X8166 and X6632 belong to the compound classes of indoles and coumarins. X8166 was shown to be superior with respect to *in vivo* studies and cytotoxicity in MEFs, while compound X6632 was more potent in inhibiting Id1/ld3 expression in the cells. Derivatives of both compound classes have been tested in further *in vitro* studies to determine their potential in comparison to the original compounds for which the *in vivo* results were obtained, and to determine the chemical space required for inhibition of Id1 and ld3 expression.

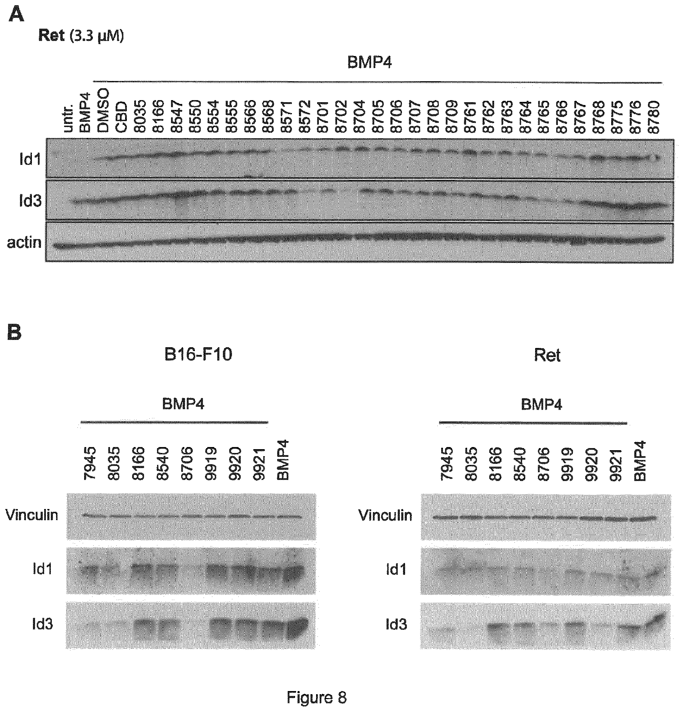

The indazole and indole-type compounds that exhibited inhibitory activity on Id1 and ld3 expression are shown in Fig. 8 and their chemical structures are described in the present application. Three closely related compound subclasses with inhibitory activity were identified.

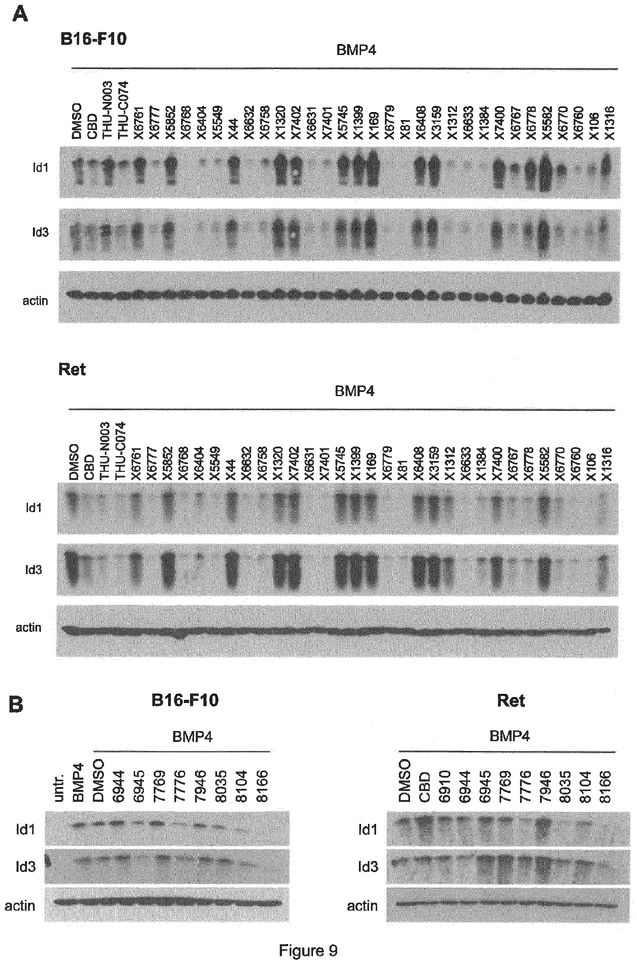

The coumarin-type compounds that exhibited inhibitory activity on Id1 and Id3 expression are shown in Figure 9 and their chemical structures are described in the present application.

### Example 5 (Reference Example):

### Synthesis of compound X6632

### 1-(3,5-Dimethoxyphenyl)cyclohexane-1-carbonitrile

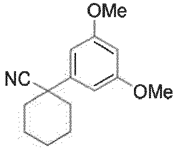

To a solution of 7.50 g of 2-(3,5-dimethoxyphenyl)acetonitrile (42.3 mmol, 1.00 equiv.) in 200 mL of abs. tetrahydrofuran under argon counterflow at -16 °C, 25.3 g of KHMDS (127 mmol, 3.00 equiv.) were added. The mixture was stirred for 3 min at the same temperature and then 6.24 mL of 1,4-dibromopentane (10.6 g, 46.6 mmol, 1.10 equiv.), diluted in 50 mL of abs. tetrahydrofuran, were added dropwise. The mixture was allowed to warm to room temperature and stirred overnight. The reaction was quenched via the addition of ammonium chloride solution (150 mL) and diluted with 100 mL of diethyl ether. The organic layers were extracted with 3 × 200 mL of diethyl ether and the combined organic layers were dried over sodium sulfate. Removal of the volatiles under reduced pressure and purification via flash column chromatography (CH/EtOAc 5:1) resulted in 8.09 g (78%) of the pure product as a colorless oil. Analytical data are consistent with the literature.

***R*_{f}** (CH/EtOAc 5:1): 0.43. - **¹H NMR** (300 MHz, CDCl₃): δ = 6.63 (d, *J* = 2.2 Hz, 2H, 2 × *H*_{Ar}), 6.40 (t, *J* = 2.2 Hz, 1H, *H*_{Ar}), 3.81 (s, 6H, 2 × OC*H*₃), 2.21 - 2.16 (m, 2H, CH₂), 1.93 - 1.65 (m, 6H, CH₂), 1.46 - 1.02 (m, 2H, C*H*₂) ppm.

### 1-(3,5-Dimethoxyphenyl)cyclohexane-1-carbaldehyde

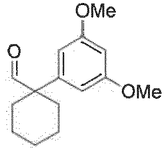

A solution of 7.97 g of 1-(3,5-dimethoxyphenyl)cyclohexane-1-carbonitrile (32.5 mmol, 1.00 equiv.) in 250 mL of abs. dichloromethane under argon atmosphere was cooled to -78 °C and 81.2 mL of DIBAL-H (1 m in dichloromethane, 81.2 mmol, 2.50 equiv.) were added dropwise. The mixture was stirred for an additional 1 h at the same temperature and then the reaction was quenched by dropwise addition of 120 mL of 10% aqueous sodium potassium-tartrate. After thawing up to room temperature the mixture was stirred for another 40 min and the aqueous layer extracted with 3 × 200 mL of ethyl acetate. The combined organic layers were washed with 300 mL of brine and dried over sodium sulfate. Removal of the volatiles under reduced pressure and purification via flash column chromatography (CH/EtOAc 10:1) resulted in 7.39 g (92%) of the pure product as a colorless oil.

***R_{f}*** (CH/EtOAc 20:1): 0.20. - **¹H NMR** (300 MHz, CDCl₃): δ = 9.34 (s, 1H, C*H*O), 6.46 (d, *J* = 2.2 Hz, 2H, 2 × *H*_{Ar}), 6.37 (t, *J* = 2.2 Hz, 1H, *H*_{Ar}), 3.78 (s, 6H, 2 × OC*H*₃), 2.27-2.22 (m, 2H, CH₂), 1.85-1.78 (m, 2H, CH₂), 1.69-1.57 (m, 3H, C*H*₂), 1.52-1.25 (m, 3H, C*H*₂) ppm.

### (Z)-1-(1-(But-1-en-1-yl)cyclohexyl)-3,5-dimethoxybenzene

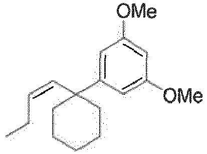

To a suspension of 36.3 g of n-propyl triphenylphosphonium bromide (94.3 mmol, 3.00 equiv.) in 300 mL of abs. tetrahydrofuran at 0 °C, 18.8 g of KHMDS (94.3 mmol, 3.00 equiv.) were added under argon counterflow. The mixture was stirred for 30 min at 10 °C and a solution of 7.81 g of 1-(3,5-dimethoxyphenyl)cyclohexane-1-carbaldehyde (33.7 mmol, 1.00 equiv.) in 50 mL of abs. tetrahydrofuran was added dropwise. After stirring for another 60 min the reaction was quenched by the addition of 200 mL of ammonium chloride solution. The aqueous layer was extracted with 3 × 200 mL of diethyl ether and the combined organic layers were dried over sodium sulfate. Removal of the volatiles under reduced pressure and purification via flash column chromatography (CH/EtOAc 10:1) resulted in 8.62 g (99%) of the pure product as a colorless oil. Analytical data are consistent with the literature.

Rf (CH/EtOAc 5:1): 0.65. - **¹H NMR** (300 MHz, CDCl₃): δ = 6.58 (d, *J* = 2.3 Hz, 2H, 2 × *H*_{Ar}), 6.28 (t, *J* = 2.3 Hz, 1H, *H*_{Ar}), 5.63 (dt, *J* = 11.2 Hz, *J* = 1.7 Hz, 1H, *H*_{DB}), 5.34 (dt, *J* = 11.2 Hz, *J* = 7.4 Hz, 1H, *H*_{DB}), 3.78 (s, 6H, 2 × OC*H*₃), 1.95-1.90 (m, 2H, C*H*₂), 1.72-1.56 (m, 9H, C*H*₂), 1.31-1.24 (m, 1H, C*H*₂), 0.72 (t, *J* = 7.5 Hz, 3H, C*H*₃) ppm.

### 1-(1-Butylcyclohexyl)-3,5-dimethoxybenzene

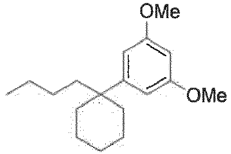

To a solution of 8.50 g of ((Z)-1-(1-(but-1-en-1-yl)cyclohexyl)-3,5-dimethoxybenzene in ethyl acetate 1.73 g of palladium on activated charcoal (10% Pd/C) were added. Hydrogen gas was bubbled through the solution for several hours and subsequently kept under hydrogen atmosphere for 24 h. Filtration through Celite^{®}, rinsing with ethyl acetate and removal of the volatiles resulted in 8.31 g (97%) of the pure product as a colorless oil. Analytical data are consistent with the literature.

***R*_{f}** (CH/EtOAc 5:1): 0.68. - **¹H NMR** (300 MHz, CDCl₃): δ = 6.48 (d, *J* = 2.3 Hz, 2H, 2 × *H*_{Ar}), 6.3 (t, *J* = 2.3 Hz, 1H, *H*_{Ar}), 3.80 (s, 6H, 2 × OC*H*₃), 2.02-1.98 (m, 2H, C*H*₂), 1.57-1.36 (m, 10H, 5 × C*H*₂), 1.18-1.09 (m, 2H, C*H*₂), 0.96-0.88 (m, 2H, C*H*₂), 0.78 (t, *J* = 7.3 Hz, 3H, C*H*₃) ppm.

### 4-(1-Butylcyclohexyl)-2,6-dimethoxybenzaldehyde

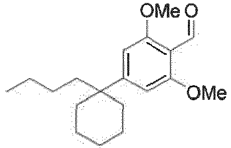

8.26 g of 1-(1-butylcyclohexyl)-3,5-dimethoxybenzene (29.9 mmol, 1.00 equiv.) were dissolved in 60 mL of diethyl ether and 6.72 mL of TMEDA (5.21 g, 44.8 mmol, 1.50 equiv.) were added dropwise. The solution was cooled to 0 °C and 17.9 mL of *n*-butyl lithium (2.5 m in n-hexanes, 44.8 mmol, 1.50 equiv.) were added slowly. After stirring for 4 h at room temperature, the solution was cooled to 0 °C, 6.89 mL of dimethylformamide (6.551 g, 89.7 mmol, 3.00 equiv.) were added and the mixture was stirred for another 4 h at room temperature. The reaction was quenched by the addition of 60 mL of brine and extracted with 3 × 15 mL of diethyl ether. The combined organic layers were dried over sodium sulfate, the volatiles were removed under reduced pressure and the residue was then purified via flash column chromatography (CH/EtOAc 10:1) to result in 8.31 g (91%) of the product as yellow oil that was used directly in the next step. Analytical data are consistent with the literature.

***R*_{f}** (CH/ EtOAc 10:1): 0.19. **- ¹H NMR** (300 MHz, CDCl₃): δ = 10.46 (s, 1H, C*H*O), 6.52 (s, 2H, 2 × H_{Ar}), 3.89 (s, 6H, 2 × OC*H*₃), 2.07-1.94 (m, 2H, C*H*₂), 1.66 - 1.33 (m, 10H, 5 × C*H*₂), 1.29-1.04 (m, 2H, C*H*₂), 1.01-0.85 (m, 2H, C*H*₂), 0.79 (t, *J* = 7.3 Hz, 3H, C*H*₃) ppm.

### 4-(1-Butylcyclohexyl)-2-hydroxy-6-methoxybenzaldehyde

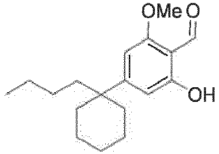

8.00 g of 4-(1-Butylcyclohexyl)-2,6-dimethoxybenzaldehyde (1.00 eq, 26.3 mmol) were dissolved in a mixture of 100 mL of dry acetonitrile and 50 mL of dry dichloromethane, cooled to 0 °C and 8.76 g of aluminum trichloride (65.7 mmol, 2.50 eq,) and 9.85 g of sodium iodide (65.7 mmol, 2.50 eq,) were added slowly under argon counterflow. The reaction mixture was stirred for 1.5 h at room temperature, quenched with water, extracted with 3 × 30 mL of dichloromethane, the combined organic layers were washed with sodium thiosulfate solution, dried over sodium sulfate and after removal of volatiles, the crude product was purified via flash column chromatography (CH/EtOAc 40:1) to result in 6.11 g (80%) of a yellow oil. Analytical data are consistent with the literature.

***R*_{f}** (CH/EtOAc 40:1): 0.26. - **¹H NMR** (300 MHz, CDCl₃): δ = 11.92 (s, 1H, C2-O*H*), 10.26 (s, 1H, C*H*O), 6.51 (d, *J* = 1.3 Hz, 1H, *H*_{Ar}), 6.34 (d, *J* = 1.3 Hz, 1H *H*_{Ar}), 3.88 (s, 3H, OC*H*₃), 2.00-1.32 (m, 12H, 6 × C*H*₂), 1.20-1.10 (m, 2H, C*H*₂), 0.96-0.88 (m, 2H, C*H*₂), 0.79 (t, *J* = 7.3 Hz, 3H, C*H*₃) ppm.

### 7-(1-Butylcyclohexyl)-5-methoxy-3-butyl-2H-chromen-2-one

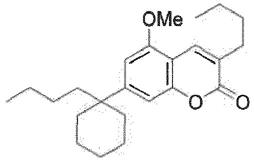

200 mg of 4-(1-Butylcyclohexyl)-2-hydroxy-6-methoxybenzaldehyde (0.69 mmol, 1.00 equiv.), 0.56 mL of hexanoic acid anhydride (517 mg, 2.41 mmol, 3.50 equiv.) and 4.8 mg of potassium carbonate (270 µmol, 0.05 equiv.) were placed in a microwave vial and heated at 180 °C for 65 min at 300 W microwave irradiation. The resulting mixture was allowed to cool to room temperature, poured onto crushed ice and the pH was adjusted to ∼7 with sodium bicarbonate. The mixture was then extracted with 3 × 50 mL of ethyl acetate and the combined organic layers were dried over sodium sulfate. Removal of the volatiles under reduced pressure and purification via flash column chromatography (CH/EtOAc 100:1) resulted in 210 mg (82%) of an off-white solid.

***R*_{f}** (CH/EtOAc 50:1): 0.31. - **MP**: 143.8 °C - **¹H NMR** (400 MHz, CDCl₃): δ = 7.81 (s, 1H, 4-C*H*), 6.90-6.86 (m, 1H, *H*_{Ar}), 6.66 (d, *J* = 1.5 Hz, 1H, *H*_{Ar}), 3.92 (s, 3H, OC*H*₃), 2.55 (t, *J* = 7.7 Hz, 2H, C*H*₂), 2.11-1.94 (m, 2H, C*H*₂), 1.70-1.30 (m, 14H, 7 × C*H*₂), 1.13 (p, *J* = 7.3 Hz, 2H, C*H*₂), 1.00-0.85 (m, 5H, C*H*₂, C*H*₃), 0.77 (t, *J* = 7.3 Hz, 3H, C*H*₃) ppm. - **¹³C NMR** (100 MHz, CDCl₃): δ = 162.4 (C_{quart.}, COO), 155.4 (C_{quart.}, *C*_{Ar}), 154.2 (C_{quart.}, *C*_{Ar}), 152.4 (C_{quart.}, *C*_{Ar}), 133.5 (+, 4-*C*_{Ar}H), 127.2(C_{quart.}, *C*_{Ar}), 108.0 (C_{quart.}, CAr), 107.9 (+, *C*_{Ar}H), 103.9 (+, *C*_{Ar}H), 55.9 (+, OC*H*₃), 43.6(C_{quart.}, *C*_{CH}), 42.3 (-, *C*H₂), 36.5 (-, 2 × *C*H₂), 30.8 (-, *C*H₂), 30.5 (-, *C*H₂), 26.6 (-, *C*H₂), 25.8 (-, *C*H₂), 23.4 (-, *C*H₂), 22.6 (-, 2 × *C*H₂), 14.1 (+, *C*H₃), 14.0 (+, *C*H₃) ppm. **- IR** (KBr): v⁻ = 2926 (m), 2854 (w), 1714 (m), 1613 (m), 1570 (w), 1495 (w), 1453 (m), 1413 (m), 1376 (w), 1344 (w), 1291 (w), 1245 (m), 1163 (w), 1104 (m), 1073 (w), 1044 (m), 991 (m), 943 (w), 906 (w), 834 (w), 798 (w), 760 (w), 712 (w), 684 (w), 653 (w), 558 (w), 494 (vw), 429 (vw) cm⁻¹. - **MS** (70 eV, El): *m*/*z* (%) = 370 (96) [M]⁺, 328 (7), 327 (8), 315 (10), 314 (45), 313 (100) [M - C₄H₉]⁺, 274 (6), 271 (5), 259 (5), 246 (10), 245 (54), 233 (19), 203 (7), 202 (11), 189 (5), 81 (7). **- HRMS** (C₂₄H₃₄O₃): calc. 370.2502, found 370.2502. - **Elemental analysis**: C₂₄H₃₄O₃: calc. C 77.80, H 9.25, found C 77.78, H 9.43.

### 7-(1-Butylcyclohexyl)-5-hydroxy-3-butyl-2H-chromen-2-one

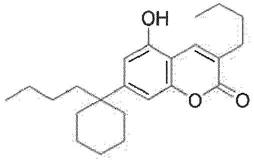

116 mg of 7-(1-Butylcyclohexyl)-5-methoxy-3-butyl-2*H*-chromen-2-one (356 µmol, 1.00 equiv.) were dissolved in 5 mL of dry dichloromethane. The solution was cooled to -78 °C and 1.78 mL of boron tribromide (1 m in dichloromethane, 1.78 mmol, 5.00 equiv.) were added dropwise. The mixture was stirred for 30 min at this temperature and then allowed to warm to room temperature. The reaction was quenched after 16 h at 0 °C by addition of sodium bicarbonate. The aqueous layer was extracted with 3 × 15 mL of dichloromethane and the combined organic layers were washed with brine, dried over sodium sulfate and the volatiles were removed under reduced pressure. The crude product was then purified via flash column chromatography (CH/EtOAc 5:1) to give the product as 116 mg (92%) of a white solid.

***R*_{f}** (CH/EtOAc 5:1): 0.43. - **MP**: 154.0 °C - **¹H NMR** (400 MHz, CDCl₃): δ = 7.89 (s, 1H, 4-C*H*), 6.83 (d, *J* = 1.4 Hz, 1H, *H*_{Ar}), 6.73 (d, *J* = 1.5 Hz, 1H, *H*_{Ar}), 6.54 (s, 1H, O*H*), 2.70-2.48 (m, 2H, C*H*₂), 2.04-1.93 (m, 2H, C*H*₂), 1.68-1.58 (m, 2H, C*H*₂), 1.57-1.27 (m, 12H, 6 × C*H*₂), 1.17-1.05 (m, 2H, C*H*₂), 0.94 (t, *J* = 7.3 Hz, 3H, C*H*₃), 0.92-0.83 (m, 2H, C*H*₂), 0.75 (t, *J* = 7.3 Hz, 3H, C*H*₃) ppm. - **¹³C NMR** (100 MHz, CDCl₃): δ = 163.3 (C_{quart.}, COO), 154.3 (C_{quart.}, *C*_{Ar}), 152.7 (C_{quart.}, *C*_{Ar}), 152.2 (C_{quart.}, *C*_{Ar}), 134.3 (+, 4-*C*_{Ar}H), 126.8 (C_{quart.}, *C*_{Ar}), 109.1 (+, *C*_{Ar}H), 107.5 (+, *C*_{Ar}H), 107.1 (C_{quart.}, *C*_{Ar}), 43.8 (-, *C*H₂), 42.0 (C_{quart.}, *C*_{CH}), 36.4 (-, 2 × *C*H₂), 30.7 (-, *C*H₂), 30.5 (-, *C*H₂), 26.6 (-, *C*H₂), 25.8 (-, *C*H₂), 23.4 (-, *C*H₂), 22.6 (-, *C*H₂), 22.5 (-, 2 × *C*H₂), 14.1 (+, *C*H₃), 14.0 (+, *C*H₃) ppm. **- IR** (KBr): v⁻ = 3171 (w), 2925 (w), 2853 (w), 1670 (m), 1613 (m),1573 (w), 1451 (w), 1421 (m), 1345 (w), 1288 (w), 1253 (w), 1185 (w), 1124 (w), 1101 (w), 1066 (w), 939 (w), 862 (w), 842 (w), 782 (w), 745 (w), 728 (w), 608 (vw), 529 (w), 414 (vw) cm⁻¹. - **MS** (70 eV, El): *m*/*z* (%) = 356 (71) [M]⁺, 331 (8), 314 (12), 301 (8), 300 (46), 299 (100) [M - C₄H₉]⁺, 281 (8), 262 (7), 260 (9). - **HRMS** (C₂₃H₃₂O₃): calc. 356.2346, found 356.2347. - **Elemental analysis:** C₂₃H₃₂O₃: calc. C 77.49, H 9.05, found C 77.27, H 9.09.

### Example 6:

### Synthesis of compound X8166

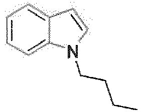

1-H Indole (1.00 g, 8.54 mmol, 1.00 equiv) was dissolved under inert atmosphere in 50 mL of dry DMF. Sodium hydride (512 mg, 12.80 mmol, 1.50 equiv) and 1-iodobutane (2.36 g, 1.46 mL, 13 mmol, 1.50 equiv) were added at 0 °C and the reaction was stirred over night at 21 °C. The workup of the reaction was done by pouring the reaction on ice and extraction of the organic phases with ethyl acetate. The combined organic layers were dried over Na₂CO₃, filtrated and the solvent was removed under reduced pressure. The crude product was purified by flash chromatography in cyclohexane:ethyl acetate 50:1. *R_{f}* = 0.73 (cyclohexane/ethyl acetate 4:1).

**¹H NMR** (400 MHz, CDCl₃, ppm) δ = 0.98 (t, *J* = 7.3 Hz, 3H), 1.39 (dq, *J* = 15.1 Hz, *J* = 7.4 Hz, 2H), 1.81-1.91 (m, 2H), 4.16 (t, *J* = 7.1 Hz, 2H), 6.53 (dd, *J* = 3.1 Hz, *J* = 0.7 Hz, 1H), 7.11-7.17 (m, 2H), 7.25 (td, *J* = 7.6 Hz, *J* = 1.1 Hz, 1H), 7.39 (dd, *J* = 8.3 Hz, *J* = 0.7 Hz, 1H), 7.68 (dt, *J* = 7.8 Hz, *J* = 0.9 Hz, 1H). **¹³C NMR** (100 MHz, CDCl₃, ppm) δ = 14.0, 20.5, 32.6, 46.4, 101.1, 109.7, 119.4, 121.2, 121.6, 128.1, 128.8, 136.2. **EI** (m/z, 70 eV, 15 °C): 173 (52), 130 (100). **HRMS** (C₁₂H₁₅N): Calcd 173.1199, Found 173.1199; **IR** (ATR, ṽ) = 3050, 2955, 2928, 2870, 1611, 1572, 1509, 1483, 1461, 1399, 1376, 1365, 1351, 1334, 1314, 1256, 1240, 1198, 1153, 1138, 1112, 1085, 1027, 1011, 942, 922, 884, 840, 762, 736, 714, 691, 606, 585, 569, 487, 464, 425, 395, 382 cm⁻¹. 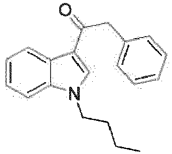 1-Butylindole (671 mg, 3.88 mmol, 1.00 equiv) was dissolved in 40 mL of abs. methylene chloride (N₂ atmosphere) and dimethylalumanylium;chloride in hexane (1 M, 4.65 mL, 4.65 mmol, 1.20 equiv) was added at 0 °C. The reaction was stirred for 15 min at 0 °C and 2-phenylacetyl chloride (1.20 g, 1.02 mL, 7.75 mmol, 1.00 equiv) was added. The reaction was allowed to come to room temperature and was stirred at 21 °C for 14 h. The work-up of the reaction was done by washing of the reaction mixture with saturated NaHCO₃ solution. The organic layer was separated, dried over Na₂SO₄ and the solvent was evaporated under reduced pressure. The crude product was purified by flash chromatography in cyclohexane:ethyl acetate (gradient: 10:1 to 4:1) to obtain the target compound in 85% yield(955 mg, 3.3 mmol). *R_{f}* = 0.41 (cyclohexane/ethyl acetate 4:1).

**¹H NMR** (400 MHz, CDCl₃, ppm) δ = 0.91 (t, *J* = 7.3 Hz, 3H), 1.30 (dq, *J* = 15.1 Hz, *J* = 7.4 Hz, 2H), 1.72-1.86 (m, 2H), 4.03-4.16 (m, 4H), 7.13-7.35 (m, 8H), 7.72 (s, 1H), 8.33-8.44 (m, 1H); **¹³C NMR** (100 MHz, CDCl₃, ppm) δ = 13.6, 20.0, 31.8, 46.9, 47.0, 109.8, 116.1, 122.6, 122.8, 123.3, 126.6, 126.7, 127.3, 128.5, 128.5,129.3,129.3,134.9,135.9,136.7,192.7; **EI** (m/z, 70 eV, 100 °C): 291 (12), 200 (100), 144 (20). **HRMS** (C₂₀H₂₁ON): Calcd 291.1618, Found 291.1617; **IR** (ATR, ṽ) = 3117, 3050, 2958, 2928, 2870, 1624, 1575, 1526, 1492, 1483, 1464, 1433, 1385, 1338, 1284, 1235, 1206, 1184, 1155, 1129, 1104, 1076, 1055, 1031, 1012, 952, 929, 915, 871, 857, 841, 800, 777, 746, 719, 697, 643, 610, 602, 579, 554, 521, 481, 429 cm⁻¹.

## Claims

1. A compound selected from the group consisting of the following compounds X8706, X8765, X8166, X8762, X8702, X8572, X8766, X8571, and X8035:

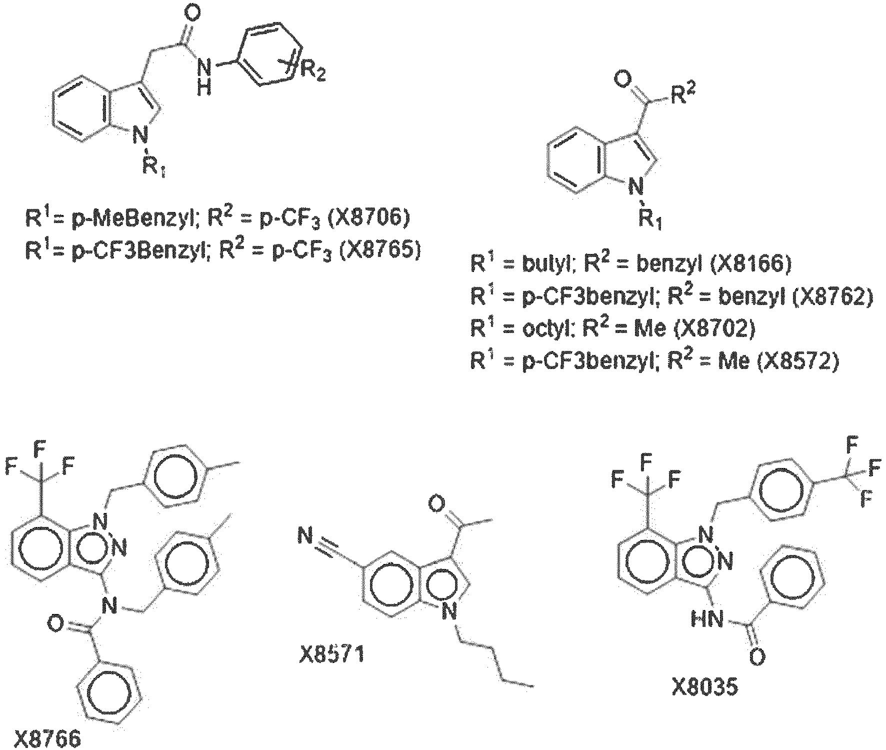

2. A compound according to claim 1 for use in medicine.

3. A compound according to claim 1 for use in the prevention or treatment of
a condition or disease that is associated with cells expressing ld1 and/or Id3,
a condition or disease that is dependent on angiogenesis, and/or
a condition or disease that is dependent on lymphangiogenesis.

4. The compound for use according to claim 3, wherein the condition or disease that is associated with cells expressing Id1 and/or ld3 is selected from the group consisting of the initiation of tumor formation, the initiation of tumor metastasis, the growth of tumors, the growth of metastases, a pro-tumor immune response, the dissemination of tumor cells, and cancer.

5. The compound for use according to claim 3, wherein the condition or disease that is dependent on angiogenesis is selected from the group consisting of the initiation of tumor formation, the initiation of tumor metastasis, the growth of tumors, the growth of metastases vascular adhesion, angiofibroma, arteriovenous malformations, arthritis, atherosclerotic plaques, corneal graft neovascularization, delayed wound healing, diabetic retinopathy, granulation burns, hemangioma, hemophilic joints, hypertrophic scars, neovascular glaucoma, non-union fractures, Osler-Weber syndrome, psoriasis, progenic granuloma, retrolental fibroplasia, schleroderma, cancer, trachoma, and von Hippel-Lindau syndrome.

6. The compound for use according to claim 3, wherein the condition or disease that is dependent on lymphangiogenesis is selected from the group consisting of organ transplantation, cancer, filariasis, Gorham's disease, dry eye disease, pulmonary fibrosis, inflammatory bowel disease, diabetes, chronic inflammatory diseases, chronic obstructive pulmonary disease (COPD), inflammatory arthritis, ulcerative colitis, psoriasis, and ocular surface diseases.

7. A compound according to claim 1 for use in the prevention or treatment of a condition or disease, selected from the group consisting of Castleman's disease, fibrodysplasia ossificans progressiva (FOP), miscarriage, and fibrosis.

8. A compound according to claim 1 for use in targeting cancer stem cells, the inhibition of angiogenesis, enhancing chemosensitivity of tumor cells, the induction of tumor cell dormancy, the maintenance of tumor cell dormancy, the inhibition of EMT (epithelial-mesenchymal transition), the suppression of VEGF-A (vascular endothelial growth factor A) expression, the suppression of VEGF-C (vascular endothelial growth factor C) expression, inducing the differentiation of stem cells and iPSCs (induced pluripotent stem cells), improving trophoblast implantation into the uterine wall, preventing an TGF-beta (transforming growth factor beta) immune-suppressive phenotype of immune cells, and/or the inhibition of myeloid-derived suppressor cells.

9. The compound for use according to any one of claims 2 to 8, wherein said compound is administered in combination with one or more further compounds, selected from the group consisting of immune checkpoint inhibitors, BRAF inhibitors, and MEK inhibitors.

## Patentansprüche

1. Verbindung, ausgewählt aus der Gruppe, bestehend aus den folgenden Verbindungen X8706, X8765, X8166, X8762, X8702, X8572, X8766, X8571 und X8035:

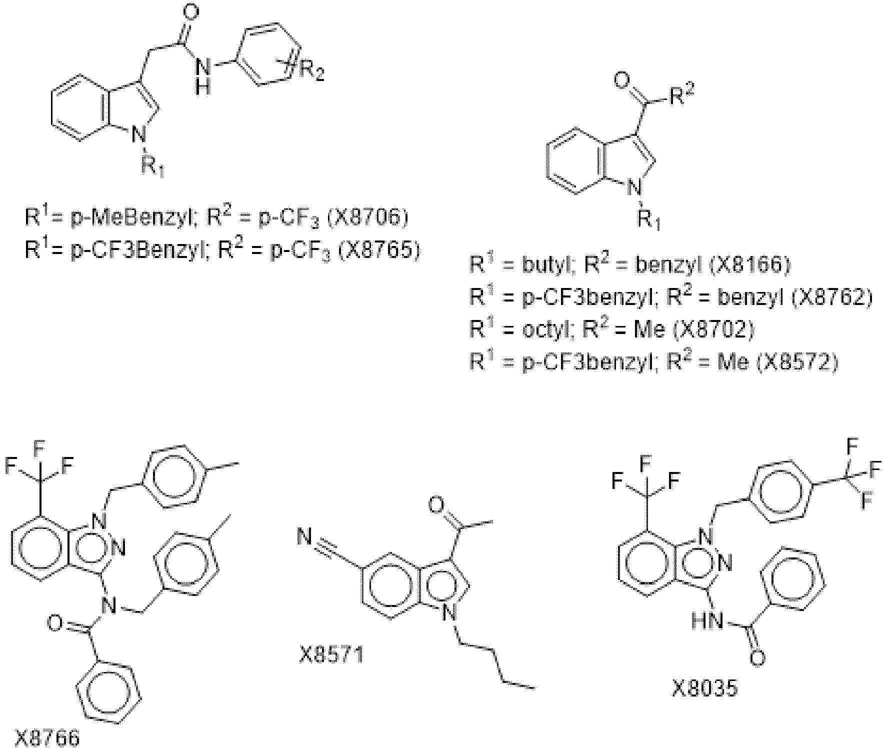

2. Verbindung nach Anspruch 1 zur Verwendung in der Medizin.

3. Verbindung nach Anspruch 1 zur Verwendung bei der Vorbeugung oder Behandlung
eines Zustands oder einer Krankheit, der/die mit Id1 und/oder Id3 exprimierenden Zellen in Verbindung steht,
eines Zustand oder einer Krankheit, der/die von der Angiogenese abhängig ist, und/oder
eines Zustands oder einer Krankheit, der/die von der Lymphangiogenese abhängig ist.

4. Verbindung zur Verwendung nach Anspruch 3, wobei der Zustand oder die Krankheit, der/die mit Id1 und/oder Id3 exprimierenden Zellen in Verbindung steht, ausgewählt ist aus der Gruppe, bestehend aus dem Einsetzen der Tumorbildung, dem Einsetzen der Tumormetastasierung, dem Wachstum von Tumoren, dem Wachstum von Metastasen, einer Pro-Tumor-Immunantwort, der Ausbreitung von Tumorzellen und Krebs.

5. Verbindung zur Verwendung nach Anspruch 3, wobei der Zustand oder die Krankheit, der/die von der Angiogenese abhängig ist, ausgewählt ist aus der Gruppe, bestehend aus dem Einsetzen der Tumorbildung, dem Einsetzen der Tumormetastasierung, dem Wachstum von Tumoren, dem Wachstum von Metastasen, vaskulärer Adhäsion, Angiofibromen, arteriovenösen Fehlbildungen, Arthritis, atherosklerotischen Plaques, Neovaskularisierung von Hornhauttransplantaten, verzögerter Wundheilung, diabetischer Retinopathie, Granulationsverbrennungen, Hämangiom, hämophilen Gelenke, hypertrophen Narben, neovaskulärem Glaukom, Nonunion-Frakturen, Osler-Weber-Syndrom, Psoriasis, progenem Granulom, retrolentaler Fibroplasie, Schlerodermie, Krebs, Trachom und von-Hippel-Lindau-Syndrom.

6. Verbindung zur Verwendung nach Anspruch 3, wobei der Zustand oder die Krankheit, der/die von der Lymphangiogenese abhängig ist, ausgewählt ist aus der Gruppe, bestehend aus Organtransplantation, Krebs, Filariose, Gorham-Krankheit, Erkrankung des trockenen Auges, Lungenfibrose, entzündlicher Darmerkrankung, Diabetes, chronisch entzündlichen Erkrankungen, chronisch obstruktiver Lungenerkrankung (COPD), entzündlicher Arthritis, Colitis ulcerosa, Psoriasis und Erkrankungen der Augenoberfläche.

7. Verbindung nach Anspruch 1 zur Verwendung bei der Vorbeugung oder Behandlung eines Zustands oder einer Krankheit, ausgewählt aus der Gruppe, bestehend aus Castleman-Krankheit, Fibrodysplasia ossificans progressiva (FOP), Fehlgeburt und Fibrose.

8. Verbindung nach Anspruch 1 zur Verwendung bei der Bekämpfung von Krebsstammzellen, der Hemmung der Angiogenese, der Steigerung der Chemosensitivität von Tumorzellen, der Induktion der Tumorzellruhe, der Aufrechterhaltung der Tumorzellruhe, der Hemmung der EMT (epithelialmesenchymaler Übergang), der Unterdrückung der Expression von VEGF-A (vaskulärer endothelialer Wachstumsfaktor A), die Unterdrückung der Expression von VEGF-C (vaskulärer endothelialer Wachstumsfaktor C), der Induktion der Differenzierung von Stammzellen und iPSCs (induzierte pluripotente Stammzellen), der Verbesserung der Einnistung von Trophoblasten in die Gebärmutterwand, der Verhinderung eines TGF-beta (transformierender Wachstumsfaktor beta) immunsuppressiven Phänotyps von Immunzellen und/oder der Hemmung von myeloiden Suppressorzellen.

9. Verbindung zur Verwendung nach einem der Ansprüche 2 bis 8, wobei die Verbindung in Kombination mit einer oder mehreren weiteren Verbindungen, ausgewählt aus der Gruppe, bestehend aus Immun-Checkpoint-Inhibitoren, BRAF-Inhibitoren und MEK-Inhibitoren, verabreicht wird.

## Revendications

1. Composé choisi parmi le groupe consistant en les composés X8706, X8765, X8166, X8762, X8702, X8572, X8766, X8571 et X8035 suivants :

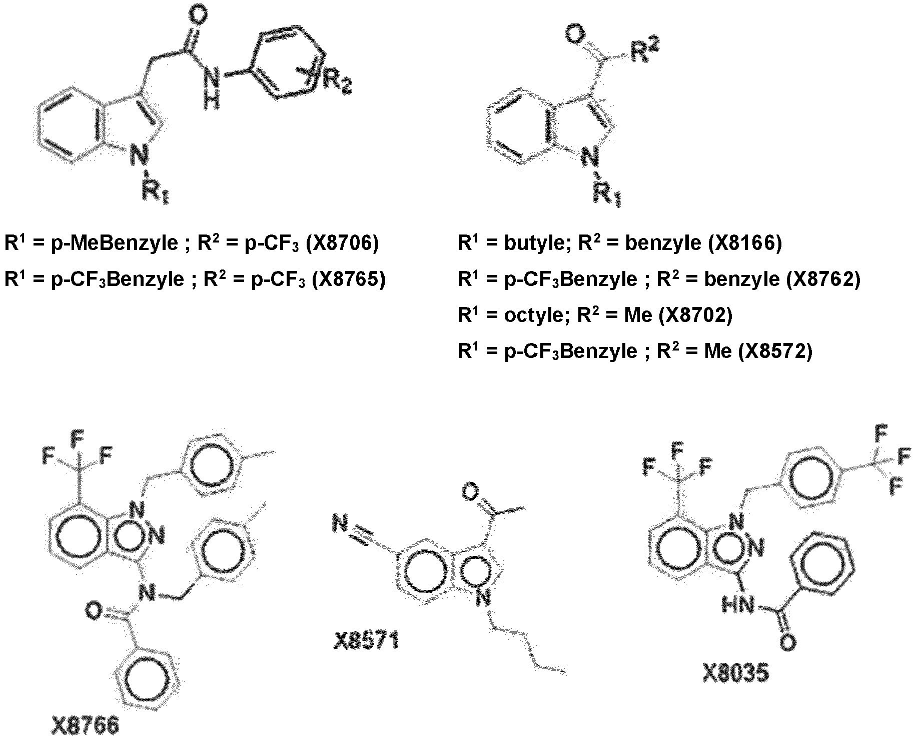

2. Composé selon la revendication 1, à utiliser en médecine.

3. Composé selon la revendication 1, à utiliser dans la prévention ou le traitement
d'un état ou d'une maladie associée à des cellules exprimant Id1 et/ou Id3,
d'un état ou d'une maladie dépendant de l'angiogenèse, et/ou
d'un état ou d'une maladie dépendant de la lymphangiogenèse.

4. Composé à utiliser selon la revendication 3, dans lequel l'état ou la maladie associée à des cellules exprimant Id1 et/ou ld3 est choisie parmi le groupe consistant en l'initiation de la formation tumorale, l'initiation de la métastase tumorale, la croissance des tumeurs, la croissance des métastases, une réponse immunitaire pré-tumorale, la dissémination des cellules tumorales et le cancer.

5. Composé à utiliser selon la revendication 3, dans lequel l'état ou la maladie dépendant de l'angiogenèse est choisie parmi le groupe consistant en l'initiation de la formation tumorale, l'initiation de la métastase tumorale, la croissance des tumeurs, la croissance des métastases adhérence vasculaire, l'angiofibrome, les malformations artérioveineuses, l'arthrite, les plaques arthérosclérotiques, la néovascularisation de la greffe de cornée, une cicatrisation retardée, la rétinopathie diabétique, la formation d'un tissu de granulation après brûlure, l'hémangiome, les articulations avec hémophilie, les cicatrices hypertrophiques, le glaucome néovasculaire, les fractures avec déplacement, le syndrome de Osler-Weber, le psoriasis, le granulome pyogénique, la fibroplasie rétrolentale, la sclérodermie, le cancer, le trachome et le syndrome de von Hippel-Lindau.

6. Composé à utiliser selon la revendication 3, dans lequel l'état ou la maladie dépendant de la lymphangiogenèse est choisie parmi le groupe consistant en la transplantation d'organe, le cancer, le filariose, la maladie de Gorham, la maladie de sécheresse oculaire, la fibrose pulmonaire, les maladies intestinales chroniques inflammatoires, le diabète, les maladies inflammatoires chroniques, la bronchopneumopathie chronique obstructive (BPCO), la polyarthrite inflammatoire, la rectocolite hémorragique, le psoriasis et les maladies de la surface oculaire.

7. Composé selon la revendication 1, à utiliser dans la prévention ou le traitement d'un état ou d'une maladie, choisie dans le groupe consistant en la maladie de Castleman, la fibrodysplasie ossifiante progressive (FOP), la fausse-couche et la fibrose.

8. Composé selon la revendication 1, à utiliser pour cibler les cellules souches cancéreuses, inhiber l'angiogenèse, augmenter la chimiosensibilité des cellules tumorales, induire la dormance des cellules tumorales, maintenir la dormance des cellules tumorales, inhiber la TEM (transition épithélial-mésenchyme), supprimer l'expression du VEGF-A (facteur de croissance endothéliale vasculaire A), supprimer l'expression du VEGF-C (facteur de croissance endothéliale vasculaire C), induire la différenciation des cellules souches et des iPCS (cellules souches pluripotentes induites), améliorer l'implantation du trophoblaste dans la paroi utérine, prévenir un phénotype immuno-suppresseur TGF-bêta (facteur de croissance transformant bêta) des cellules immunitaires, et/ou inhiber les cellules suppresseur dérivées de myéloïde.

9. Composé à utiliser selon l'une quelconque des revendications 2 à 8, dans lequel ledit composé est administré en association avec un ou plusieurs composés supplémentaires, choisis dans le groupe consistant en des inhibiteurs de point de contrôle immunitaire, des inhibiteurs de BRAF et des inhibiteurs de MEK.
